# FASCICULE DE BREVET EUROPEEN

(11) **EP 0 796 614 B1**
(45) Date de publication et mention de la délivrance du brevet: **14.03.2001**
(21) Numéro de dépôt: 97400283.4
(22) Date de dépôt: 07.02.1997
(51) Int. Cl.: A61K 7/50, A61K 7/48, A61K 7/06

(54) **Compositions cosmétiques détergentes contenant un polyacrilamide épaississant**
Kosmetische Tensidzusammensetzungen mit Acrylamid als Verdickungsmittel
Cosmetic detergent compositions containing a polyacrylamide thickener

(30) Priorité: 21.03.1996 FR 9603542
(43) Date de publication de la demande: 24.09.1997
(73) Titulaire: L'OREAL, 75008 Paris (FR)
(72) Inventeur: Dubief, Claude, 78150 Le Chesnay (FR); Cauwet-Martin, Danièle, 75011 Paris (FR)
(74) Mandataire: Andral, Christophe André Louis

(56) Documents cités:
- EP-A- 0 231 997
- EP-A- 0 494 022
- EP-A- 0 577 526
- WO-A-94/27574
- WO-A-97/16168
- WO-A-97/26860
- FR-A- 2 694 494
- GB-A- 2 136 689

## Description

La présente invention concerne de nouvelles compositions cosmétiques destinées au nettoyage des cheveux, du cuir chevelu et/ou de la peau contenant au moins un tensioactif détergent, au moins un cotensioactif, au moins un électrolyte et au moins un polyacrylamide épaississant, ainsi que leur utilisation dans cette application cosmétique.

Pour le nettoyage des cheveux et/ou de la peau, l'utilisation de compositions cosmétiques détergentes (shampooings ou gels-douche par exemple) contenant à la fois des agents tensioactifs à pouvoir lavant et un ou plusieurs agents de conditionnement est courante.

En effet, pour améliorer les propriétés cosmétiques des compositions détergentes, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est souvent nécessaire d'introduire dans ces dernières des agents de conditionnement cosmétiques comme par exemple des silicones, qui apportent alors aux cheveux traités une facilité de démêlage et de coiffage, ainsi qu'une douceur et une brillance nettement accrues.

De même, il peut être intéressant de traiter le cuir chevelu avec des compositions contenant des actifs tels que des agents anti-pelliculaires.

Compte tenu du caractère insoluble de la plus part des agents de conditionnement ou de certains agents anti-pelliculaires dans les milieux aqueux utilisés dans les shampooings, on cherche à les maintenir sous forme dispersée. Cette forme dispersée permet aux agents de conditionnement ou anti-pelliculaires de se déposer sur les cheveux ou sur la peau et de ne pas être totalement éliminés lors du rinçage. Il importe toutefois que la mise en suspension ne perturbe pas les propriétés détergentes et moussantes de la composition cosmétique.

Il existe à ce jour peu de moyens pour maintenir efficacement en suspension les agents insolubles, car il s'agit là d'un problème difficile à résoudre ; à cet effet, on a déjà proposé l'utilisation des dérivés d'esters ou d'éthers à longue chaîne ou des polysaccharides tels que la gomme de xanthane. Cependant, les esters ou éthers à longue chaîne présentent des problèmes de cristallisation qui entraînent une évolution (augmentation) de la viscosité des compositions au cours du temps ; les polysaccharides gélifiants présentent également des inconvénients, à savoir, d'une part que la mousse des compositions détergentes les contenant se développe difficilement (mauvais démarrage de mousse) et que, d'autre part, les compositions n'ont pas une texture lisse et s'écoulent par paquets, ce qui n'est pas apprécié des utilisateurs.

On a également proposé dans la demande de brevet français n° 2694494 des compositions contenant en suspension des particules non hydrosolubles contenant un tensioactif anionique, un cotensioactif non ionique ou amphotère et un électrolyte, lesdits tensioactifs étant en une quantité telle que la composition possède un comportement pseudoplastique avec un seuil d'écoulement supérieur à 0,2 Pa et présente une structure de phase lamellaire incluant des sphérulites aptes à maintenir en suspension des particules non hydrosolubles.
Cependant, ces compositions, qui permettent la mise en suspension de particules, présentent des propriétés d'usage non satisfaisantes notamment lorsqu'elles sont utilisées comme shampooing ou gel-douche. En particulier, les propriétés moussantes, telles que le démarrage de la mousse, ne donnent pas entière satisfaction. Les mousses sont généralement trop compactes et difficiles à travailler.

La demanderesse a donc cherché à améliorer les propriétés moussantes de ces compositions, tout en gardant de bonnes propriétés d'usage telles que l'aspect et la prise en main de la composition. En effet, il est préférable d'avoir des compositions ayant une viscosité suffisante pour être facilement prélevées du récipient.

La présente invention a donc pour but de proposer des compositions cosmétiques détergentes possédant de bonnes propriétés moussantes, aptes à maintenir en suspension des agents de conditionnement non hydrosolubles et présentant une viscosité suffisante.

Ainsi, la demanderesse a maintenant trouvé que ces objectifs étaient atteints en introduisant un polyacrylamide épaississant dans un milieu comprenant au moins un tensioactif anionique, au moins un cotensioactif non ionique ou amphotère, dans un rapport pondéral cotensioactif / tensioactif anionique inférieur ou égal ou 1 et au moins un électrolyte, lesdits composés étant en quantité telle que la composition présente :
(a) un comportement rhéologique en écoulement caractérisé par un domaine de contraintes pour lesquelles la viscosité est constante, suivi d'un domaine de contraintes pour lequel la viscosité diminue lorsque la contrainte augmente, et
(b) une structure de phase lamellaire, cette phase étant apte à maintenir en suspension des particules non hydrosolubles éventuellement présentes dans la composition.

Les propriétés moussantes des compositions, telles que le démarrage de la mousse, sont nettement améliorées et les compositions présentent une viscosité suffisante.
Les compositions selon l'invention sont stables, elles permettent de maintenir en suspension des particules non hydrosolubles.

L'invention a donc pour objet une composition cosmétique détergente caractérisée par le fait qu'elle comprend au moins un tensioactif anionique, au moins un cotensioactif non ionique ou amphotère, dans un rapport pondéral cotensioactif / tensioactif anionique inférieur ou égal ou 1, au moins un polyacrylamide épaississant et au moins un électrolyte présent à une concentration comprise entre 2 et 15% en poids par rapport au poids total de la composition, et choisi parmi :
i) les nitrates de lithium, de strontium, de barium, d'yttrium, de néodyme, de gadolinium, de manganèse, de zinc ou de magnésium,
ii) les chlorures de lithium, de strontium, de barium, d'yttrium, de néodyme, de gadolinium, de manganèse, de zinc, de magnésium ou de sodium,
iii) les sulfates de calcium, de strontium ou de magnésium,
iv) les acétates de strontium, de manganèse ou de magnésium,
lesdits composés étant en quantité telle que la composition présente :
(a) un comportement rhéologique en écoulement caractérisé par un domaine de contraintes pour lesquelles la viscosité est constante, suivi d'un domaine de contraintes pour lequel la viscosité diminue lorsque la contrainte augmente, et
(b) une structure de phase lamellaire, cette phase étant apte à maintenir en suspension des particules non hydrosolubles éventuellement présentes dans la composition.

De plus, les compositions présentent des propriétés lavantes et des propriétés cosmétiques avantageuses (douceur, démêlage, coiffage). Les compositions présentent enfin une texture non filante et fondante.

L'invention a également pour objet un procédé cosmétique pour le nettoyage des cheveux, de la peau et/ou du cuir chevelu à l'aide des compositions ci-dessus.

Le comportement rhéologique en écoulement des compositions est caractérisé à l'aide d'un rhéomètre à contrainte imposée (Carrimed CSHR100). Les mesures sont réalisées à 25°C en utilisant un corps de mesure plan-cone de 2 degrés d'angle et de 6 cm de diamètre.

Les contraintes, pour lesquelles la viscosité d'une composition donnée est constante, sont variables. Généralement, selon la présente invention, elles sont comprises entre 0,001 et 10 Pa et de préférence entre 0,01 et 2 Pa.

Les compositions selon l'invention présentent une phase lamellaire, c'est à dire une phase solide hydratée ou de cristaux liquides dans laquelle plusieurs bicouches sont disposées en réseau parallèle, séparées par des couches d'eau ou de solution aqueuse.
La phase lamellaire peut éventuellement contenir des sphérulites qui sont des vésicules plurilamellaires constituées de plusieurs couches de tensioactifs disposées concentriquement et de dimensions comprises entre 0,1 et 50 micromètres.

Ainsi, à titre d'exemple de tensioactifs anioniques utilisables, seuls ou mélanges, dans le cadre de la présente invention, on peut citer notamment les sels (en particulier sels alcalins, notamment de sodium, sels d'ammonium, sels d'amines, sels d'aminoalcools ou sels de magnésium) des composés suivants : les alkylsulfates, les alkyléthersulfates, les alkylamidoéthersulfates, les monoglycérides sulfates, les alkylglycérylsulfonates, les alkylsulfonates, les alkylphosphates, les alkylamidesulfonates, les alkylarylsulfonates, les α-oléfinesulfonates, les alkylsulfosuccinates, les alkyléthersulfosuccinates, les alkylamidesulfosuccinates, les alkylsulfosuccinamates, les alkylsulfoacétates, les alkylétherphosphates, les acyliséthionates, les N-acylaminoacides tels que les N-acylsarcosinates, les N-acylglutamates et les N-acyltaurates. Parmi les tensioactifs anioniques encore utilisables, on peut également citer les sels d'acides gras tels que les sels des acides undécylénique, oléique, ricinoléique, palmitique, stéarique, les acides d'huile de coprah ou d'huile de coprah hydrogénée ; les acylhydroxyacides tels que les acyl-lactylates. On peut également utiliser des tensioactifs faiblement anioniques, comme les acides alkyl-D-galactoside uroniques et leurs sels ainsi que les acides éthers carboxyliques polyoxyalkylénés, en particulier ceux comportant de 2 à 24 groupements oxyde d'éthylène, et leurs mélanges. Le radical alkyle ou acyle de tous ces différents composés comporte de préférence de 8 à 22 atomes de carbones.

A titre de cotensioactifs non ioniques utilisables selon l'invention, on peut citer les alcools, les alphadiols, les alkylphénols ou les acides gras éthoxylés, propoxylés ou glycérolés, ces composés ayant une chaîne grasse comportant de 8 à 28 atomes de carbone, le nombre de groupements d'oxyde d'éthylène ou de propylène pouvant aller de 1 à 50 et celui de glycérol notamment de 1 à 30. On peut également citer les copolymères d'oxyde d'éthylène et de propylène, les condensats d'oxyde d'éthylène et de propylène sur des alcools gras; les amines ou les amides gras polyéthoxylés ayant de préférence de 2 à 30 moles d'oxyde d'éthylène, les amides gras polyglycérolés comportant en moyenne 1 à 5 groupements glycérol, les diglycolamides polyglycérolés, les esters d'acides gras du sorbitan éventuellement oxyéthylénés, les esters d'acides gras du sucrose, les esters d'acides gras polyoxyalkylénés, les alkylpolyglycosides éventuellement oxyalkylénés, les esters d'alkylglucosides, les dérivés de N-alkylglucamine et de N-acylrnéthylglucamines, les oxydes d'amine et leurs mélanges.

A titre de cotensioactifs amphotères utilisables selon l'invention, on peut citer les dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer les alkylbétaines, les alkyldiméthylbétaines, les alkylsulfobétaines, les alkylamidoalkylbétaines, les alkylamidoalkylsulfobétaines, les dérivés d'imidazoline tels que les dérivés d'amphocarboxyglycinate ou d'ampho-carboxypropionate et leurs mélanges.
On peut également utiliser des mélanges de cotensioactifs.

Les cotensioactifs préférés selon l'invention sont choisis parmi les alcools gras éthoxylés et les dérivés de bétaïne.

Le ou les tensioactifs anioniques sont généralement présents dans les compositions conformes à l'invention dans des proportions comprises entre 3 à 50 % en poids, de préférence de 5 à 30 % en poids, par rapport au poids total de la composition.

Le ou les cotensioactifs sont généralement présents dans les compositions conformes à l'invention dans des proportions comprises entre 0,05 et 20 % en poids, de préférence de 1 à 15 % en poids, par rapport au poids total de la composition.

La somme des concentrations en tensioactifs anioniques et cotensioactifs est généralement comprise entre 3 et 70 % en poids par rapport au poids total de la composition.

Le rapport pondéral cotensioactif / tensioactif anionique plus particulièrement compris entre 0,01 et 1 et préférentiellement compris entre 0,05 et 0,75.

Parmi les électrolytes, on peut citer les sels métalliques.

Les sels métalliques sont plus particulièrement choisis parmi les sels de métaux alcalins, de métaux alcalino-terreux, de métaux de transition et de métaux des groupes 13 et 14 de la classification périodique des éléments.
Comme sels de métaux alcalins utiles selon l'invention, on peut citer en particulier les sels de lithium, de sodium ou de potassium.
Comme sels de métaux alcalino-terreux utiles selon l'invention, on peut citer en particulier les sels de béryllium, de magnésium, de calcium, de strontium et/ou de baryum.
Comme sels de métaux de transition utiles selon l'invention, on peut citer en particulier les sels de lanthanides, et les sels de métaux de la quatrième période de la classification périodique des éléments, comme les sels de manganèse, de cobalt ou de zinc.
Comme sels de métaux des groupes 13 et 14 de la classification périodique des éléments utiles selon l'invention, on peut citer les sels d'aluminium et d'étain.
Par lanthanide, on entend les éléments de numéro atomique z allant de 57 à 71, c'est-à-dire le lanthane, le cérium, le praséodyme, le néodyme, le prométhium, le samarium, l'europium, le gadolinium, le terbium, le dysprosium, l'holmium, l'erbium, le thulium, l'ytterbium, le lutétium.
De manière préférentielle, les sels métalliques selon l'invention sont choisis parmi les sels de lithium, de strontium, de barium, d'yttrium, de néodyme, de gadolinium, de manganèse et de zinc, plus préférentiellement de strontium.
De préférence le sel est choisi parmi les nitrates ou les chlorures, en particulier le nitrate de lithium, de strontium, de barium, d'yttrium, de néodyme, de gadolinium, de manganèse ou de zinc, le chlorure de lithium, de strontium, de barium, d'yttrium, de néodyme, de gadolinium, de manganèse ou de zinc, les sulfates ou les acétates, comme le sulfate de calcium, de strontium ou de magnésium et l'acétate de strontium ou de magnésium.

De façon encore plus avantageuse, l'électrolyte est un sel de magnésium ou de strontium, se présentant notamment sous forme de chlorure ou de nitrate.

Selon l'invention, la concentration en électrolyte est de préférence inférieure à 20 % en poids par rapport au poids total de la composition et plus particulièrement comprise entre 2 et 15 %.

Les polyacrylamides épaississants selon l'invention sont de préférence les polyacrylamides dont la solution dans l'eau à 0,1% en poids présente une viscosité supérieure à environ 5 mPa.s (mesurée à 25°C avec un viscosimètre Contraves TV module 1).

Les polyacrylamides épaississants peuvent plus particulièrement être choisis parmi :
- les copolymères d'acrylamide et d'acrylate d'ammonium éventuellement réticulés,
- les copolymères d'acrylamide (ou de méthacrylamide) et d'halogènure ( par exemple chlorure) de méthacryloyloxyéthyl triméthylammonium éventuellement réticulés,
- les copolymères d'acrylamide et d'acide 2-acrylamido 2-méthyl propanesulfonique partiellement ou totalement neutralisés éventuellement réticulés.

Le copolymère réticulé d'acrylamide / acrylate d'ammonium, utilisé conformément à la présente invention, est plus particulièrement un copolymère acrylamide / acrylate d'ammonium (5/95 en poids) réticulé par un agent de réticulation à polyinsaturation oléfinique, tel que le divinylbenzène, le tétraallyloxyéthane, le méthylène bis-acrylamide, l'éther diallylique, des éthers polyallylpolyglycéryliques ou les éthers allyliques d'alcool de la série des sucres, tels que l'érythritol, le pentaérythritol, l'arabitol, le mannitol, le sorbitol ou le glucose.

Des copolymères analogues sont décrits et préparés dans le brevet français FR-2.416.723 et les brevets US-2.798.053 et US-2.923.692.

On utilise en particulier ce copolymère réticulé sous forme d'émulsion eau-dans-huile constituée d'environ 30 % en poids dudit copolymère, 25 % en poids d'huile de paraffine, 4 % en poids de mélange de stéarate de sorbitan et d'un dérivé éthoxylé hydrophile et 41 % en poids d'eau. Une telle émulsion est commercialisée sous la dénomination "BOZEPOL C" par la Société HOECHST.

Les copolymères d'acrylamide et de l'acide 2-acrylamido 2-méthyl propane sulfonique, utilisés conformément à la présente invention, sont des copolymères réticulés par un composé à polyinsaturation oléfinique, tels que ceux évoqués précédemment, et partiellement ou totalement neutralisés par un agent de neutralisation tel que la soude, la potasse, l'ammoniaque ou une amine telle que la triéthanolamine ou la monoéthanolamine.

Ils peuvent être préparés en copolymérisant l'acrylamide et le 2-acrylamido 2-méthylpropane sulfonate de sodium par voie radicalaire au moyen d'agents initiateurs du type azobisisobutyronitrile et par précipitation dans un alcool tel que le tertiobutanol.

On utilise plus particulièrement des copolymères obtenus par copolymérisation de 70 à 55 % en moles d'acrylamide et de 30 à 45 % en moles de 2-acrylamido 2-méthylpropane sulfonate de sodium. L'agent de réticulation étant utilisé à des concentrations de 10⁻⁴ à 4.10⁻⁴ mole par mole du mélange de monomères.

Ces copolymères particuliers sont incorporés dans les compositions de l'invention, de façon préférentielle, sous forme d'émulsions eau dans l'huile contenant de 35 à 40 % en poids de ce copolymère, de 15 à 25 % en poids d'un mélange d'hydrocarbures isoparaffiniques en C₁₂-C₁₃, de 3 à 8 % en poids de lauryléther de polyéthylèneglycol à 7 moles d'oxyde d'éthylène et d'eau. Une telle émulsion est commercialisée sous le nom de "SEPIGEL 305" par la société SEPPIC.

Le copolymère d'acrylamide et de chlorure de méthacryloyl oxyéthyl triméthylammonium réticulé, utilisé selon l'invention, est plus particulièrement un copolymère obtenu par copolymérisation de l'acrylamide et du diméthylaminoéthylméthacrylate quaternisé par le chlorure de méthyle, suivie d'une réticulation par un composé à insaturation oléfinique, en particulier le méthylène-bis acrylamide.

On utilise plus particulièrement un copolymère réticulé acrylamide/chlorure de méthacryloyl oxyéthyl triméthylammonium (environ 50/50 en poids) sous forme de dispersion contenant 50 % en poids dudit copolymère dans de l'huile minérale. Cette dispersion est commercialisée sous le nom de "SALCARE SC92" par la Société ALLIED COLLOIDS.

Les copolymères non réticulés de méthacrylamide et de chlorure de méthacryloyloxyéthyl triméthylammonium sont par exemple les produits vendus sous les dénominations commerciales ROHAGIT KF 400 et KF720 par la société ROHM

Les polyacrylamides épaississants sont généralement présents dans les compositions selon l'invention dans des concentrations comprises entre 0,05 et 5% en poids, et de préférence entre 0,2 et 3 % en poids par rapport au poids total de la composition.

Les compositions selon l'invention présentent généralement une viscosité supérieure à 300 mPa.s.

Par particules non hydrosolubles, on désigne des entités, solides ou non, qui ne se solubilisent pas dans le milieu aqueux de la composition.

Les particules non hydrosolubles pouvant être dispersées dans les compositions selon l'invention sont par exemple les gommes, les résines ou les huiles de silicones modifiées ou non, les composés fluorés, les agents antipelliculaires, les huiles végétales, minérales ou synthétiques, les cires, les agents nacrants, les pigments, les esters d'acide gras, les particules abrasives telles que la silice, les parfums et les polymères insolubles dans l'eau.

Les compositions détergentes selon l'invention présentent un pH final généralement compris entre 3 et 8. De préférence, ce pH est compris entre 4 et 7,5. L'ajustement du pH à la valeur désirée peut se faire classiquement par ajout, selon les cas, soit d'agents basifiants, soit d'agents acidifiants, usuels et connus comme cosmétiquement acceptables.

Les compositions détergentes selon l'invention peuvent bien entendu contenir en outre tous les adjuvants habituellement rencontrés dans le domaine des compositions détergentes pour les cheveux et/ou pour le corps, comme par exemple des parfums, des agents conservateurs, des séquestrants, des agents acidifiants, des agents alcalinisants, des adoucissants, des modificateurs de mousse, des colorants, des agents nacrants, des agents hydratants, des agents antipelliculaires ou anti-séborrhéiques, des vitamines, des silicones, des céramides, des filtres solaires, des polymères de préférence cationiques ou amphotères et autres.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Ces compositions peuvent se présenter sous la forme de liquides épaissis, de crèmes ou de gels et elles conviennent principalement au lavage des cheveux et/ou de la peau.

L'invention a encore pour objet un procédé de lavage de la peau ou des fibres kératiniques, telles que les cheveux, consistant à appliquer sur ceux-ci une composition telle que définie précédemment, puis à effectuer un rinçage à l'eau.

Des exemples concrets illustrant l'invention vont maintenant être donnés. Dans ce qui suit MA signifie matière active.

### EXEMPLE 1

On a préparé un shampooing conforme à l'invention qui présente la composition suivante :
- Lauryl éther sulfate de sodium à 2,2 moles d'oxyde d'éthylène en solution aqueuse à 28 % de MA 11,2 g MA
- Oléylamidopropyl diméthyl bétaïne en solution aqueuse à 28 % de MA 8,4 g MA
- Disulfure de sélénium 0,5 g
- NaCl 3 g
- Polyacrylamide en émulsion inverse à 40% MA (SEPIGEL 305 de SEPPIC) 1 g MA
- Conservateurs, colorants, parfum qs
- HCl qs pH 6,5
- Eau qsp 100 g

Ce shampooing se présente sous forme d'un lait épais. Il est appliqué sur les cheveux mouillés et on obtient rapidement une mousse onctueuse et agréable.
Il présente de bonnes propriétés moussantes et apporte aux cheveux douceur et facilité de démêlage.

La composition présente un profil d'écoulement avec un domaine de contraintes pour lesquelles la viscosité est constante suivi d'un domaine de contraintes pour lesquelles la viscosité diminue lorsque la contrainte augmente. L'observation de la composition au microscope optique ou électronique indique une structure lamellaire.
La composition est stable, le disulfure de sélénium est maintenu en suspension.

### EXEMPLE 2

On a préparé un shampooing conforme à l'invention qui présentait la composition suivante :
- Lauryl éther sulfate de sodium à 2,2 moles d'oxyde d'éthylène en solution aqueuse à 28 % de MA 21 g MA
- Alcool (C12-C15) polyoxypropyléné à 3 moles d'oxyde de propylène 3 g
- Polydiméthylsiloxane(PDMS)(Huile 47 V 500.000 RHONE POULENC) 3 g
- NaCl 10 g
- Polyquaternium-32 (CTFA) en émulsion inverse à 50% MA (SALCARE SC 92 de ALLIED COLLOID) 0,5 g MA
- Conservateurs, colorants, parfum qs
- NaOH qs pH 6,8
- Eau qsp 100 g

Ce shampooing se présente sous forme d'un lait crémeux. Il présente de bonnes propriétés moussantes et apporte aux cheveux douceur et facilité de démêlage.

La composition présente un profil d'écoulement avec un domaine de contraintes pour lesquelles la viscosité est constante suivi d'un domaine de contraintes pour lesquelles la viscosité diminue lorsque la contrainte augmente. L'observation de la composition au microscope optique ou électronique indique une structure lamellaire.
La composition est stable, la silicone est maintenue en suspension.

### EXEMPLE 3 (Invention)

On a préparé un shampooing conforme à l'invention qui présentait la composition suivante :
- Lauryl éther sulfate de sodium à 2,2 moles d'oxyde d'éthylène en solution aqueuse à 28 % de MA 16,6 g MA
- Oléylamidopropyl diméthyl bétaïne en solution aqueuse à 28 % de MA 2,7 g MA
- Polydiméthylsiloxane(PDMS)(Huile 47 V 500.000 RHONE POULENC) 2,7 g
- Diméthicone copolyol vendu sous la dénomination DC 190 par la société DOW CORNING 1 g
- NaCl 12 g
- Polyacrylamide en émulsion inverse à 40% MA (SEPIGEL 305 de SEPPIC) 1 g MA
- Conservateurs, colorants, parfum qs
- HCl qs pH 6,5
- Eau qsp 100 g

Ce shampooing se présente sous forme d'un lait crémeux. Il présente de bonnes propriétés moussantes et apporte aux cheveux douceur et facilité de démêlage.

La composition présente un profil d'écoulement avec un domaine de contraintes pour lesquelles la viscosité est constante suivi d'un domaine de contraintes pour lesquelles la viscosité diminue lorsque la contrainte augmente. L'observation de la composition au microscope optique ou électronique indique une structure lamellaire. La composition présente une viscosité d'environ 1500 mPa.s (mesurée à 25°C avec un viscosimètre Contraves TV module 3).

La composition est stable, les silicones sont maintenues en suspension.

### EXEMPLES 4, 5, 6, 7 (COMPARATIFS)

On a préparé des shampooings similaires à celui de l'exemple 3 dans lequel on a remplacé le polyacrylamide par la même quantité d'un autre épaississant :
Exemple 4 : Gomme de xanthane
Exemple 5 : Copolymère acrylique réticulé (CARBOPOL 1342 de GOODRICH)
Exemple 6 : Hydroxyéthylcellulose (NATROSOL 250 HHR de NATIONAL STARCH)
Exemple 7 : Copolymère de méthylvinyléther et d'anhydride maléïque réticulé (STABILEZE 06 de ISP)

On a évalué le pouvoir moussant de ces compositions à l'aide d'un panel de testeurs expérimentés sur les critères de développement et d'abondance de mousse. On applique 1,5 g de chaque composition sur des mèches de 5 g de cheveux mouillés à l'eau et essorés par passage entre les doigts. Le testeur malaxe chaque mèche avec les mains propres et humides pour développer la mousse.

La composition 3 selon l'invention présente un excellent pouvoir moussant. Les compositions 4 à 7 ont un pouvoir moussant très inférieur à celui de la composition 3. Le développement de la mousse est moins rapide et la mousse obtenue est moins abondante.

Seule la composition de l'exemple 3 selon l'invention a un pouvoir moussant satisfaisant.

D'autre part, les compositions 4 à 7 sont instables : au bout d'un mois de stockage à 45°C, il y a séparation de phases, la silicone relargue en surface, la silicone n'est pas maintenue en suspension.

## Revendications

1. Composition cosmétique détergente caractérisée par le fait qu'elle comprend au moins un tensioactif anionique, au moins un cotensioactif non ionique ou amphotère, dans un rapport pondéral cotensioactif / tensioactif anionique inférieur ou égal ou 1, au moins un polyacrylamide épaississant et au moins un électrolyte présent à une concentration comprise entre 2 et 15% en poids par rapport au poids total de la composition, et choisi parmi :
i) les nitrates de lithium, de strontium, de barium, d'yttrium, de néodyme, de gadolinium, de manganèse, de zinc ou de magnésium,
ii) les chlorures de lithium, de strontium, de barium, d'yttrium, de néodyme, de gadolinium, de manganèse, de zinc, de magnésium ou de sodium,
iii) les sulfates de calcium, de strontium ou de magnésium,
iv) les acétates de strontium, de manganèse ou de magnésium,
lesdits composés étant en quantité telle que la composition présente :
(a) un comportement rhéologique en écoulement caractérisé par un domaine de contraintes pour lesquelles la viscosité est constante, suivi d'un domaine de contraintes pour lequel la viscosité diminue lorsque la contrainte augmente, et
(b) une structure de phase lamellaire, cette phase étant apte à maintenir en suspension des particules non hydrosolubles éventuellement présentes dans la composition.

2. Composition selon la revendication 1, caractérisée en ce que les tensioactifs anioniques utilisables sont choisis parmi les sels des composés suivants :
les alkylsulfates, les alkyléthersulfates, les alkylamidoéthersulfates, les monoglycérides sulfates, les alkylglycérylsulfonates, les alkylsulfonates, les alkylphosphates, les alkylamidesulfonates, les alkylarylsulfonates, les α-oléfinesulfonates, les alkylsulfosuccinates, les alkyléthersulfosuccinates, les alkylamidesulfosuccinates, les alkylsulfosuccinamates, les alkylsulfoacétates, les alkylétherphosphates, les acyliséthionates, les N-acylaminoacides tels que les N-acylsarcosinates, les N-acylglutamates et les N-acyltaurates, les sels d'acides gras tels que les sels des acides undécylénique, oléique, ricinoléique, palmitique, stéarique, les acides d'huile de coprah ou d'huile de coprah hydrogénée, les acylhydroxyacides tels que les acyl-lactylates, les acides alkyl-D-galactoside uroniques et leurs sels, les acides éthers carboxyliques polyoxyalkylénés, en particulier ceux comportant de 2 à 24 groupements oxyde d'éthylène, et leurs mélanges.

3. Composition selon l'une des revendications 1 ou 2, caractérisée en ce que les cotensioactifs non ioniques sont choisis parmi les alcools, les alphadiols, les alkylphénols ou les acides gras éthoxylés, propoxylés ou glycérolés, ces composés ayant une chaîne grasse comportant de 8 à 28 atomes de carbone, le nombre de groupements d'oxyde d'éthylène ou de propylène pouvant aller de 1 à 50 et celui de glycérol notamment de 1 à 30, les copolymères d'oxyde d'éthylène et de propylène, les condensats d'oxyde d'éthylène et de propylène sur des alcools gras; les amines ou les amides gras polyéthoxylés ayant de préférence de 2 à 30 moles d'oxyde d'éthylène, les amides gras polyglycérolés comportant en moyenne 1 à 5 groupements glycérol, les diglycolamides polyglycérolés, les esters d'acides gras du sorbitan éventuellement oxyéthylénés, les esters d'acides gras du sucrose, les esters d'acides gras polyoxyalkylénés, les alkylpolyglycosides éventuellement oxyalkylénés, les esters d'alkylglucosides, les dérivés de N-alkylglucamine et de N-acylméthylglucamines, les oxydes d'amine et leurs mélanges.

4. Composition selon l'une quelconque des revendications 1 ou 2, caractérisée en ce que les cotensioactifs amphotères sont choisis parmi les dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant carboxylate, sulfonate, sulfate, phosphate ou phosphonate.

5. Composition selon la revendication 4, caractérisée en ce que les cotensioactifs amphotères sont choisis parmi les alkylbétaines, les alkyldiméthylbétaines, les alkyl sulfo bétaines, les alkyl amido alkyl bétaines, les alkyl amido alkyl sulfobétaines, les dérivés d'imidazoline tels que les dérivés d'amphocarboxyglycinate ou d'ampho-carboxypropionate et leurs mélanges.

6. Composition selon l'une quelconque des revendications précédentes, caractérisées par le fait que le ou les tensioactifs anioniques sont présents à raison de 3 à 50% en poids par rapport au poids total de la composition, de préférence à raison de 5 à 30 % en poids.

7. Composition selon l'une quelconque des revendications précédentes, caractérisée par le fait que le ou les cotensioactifs sont présents à raison de 0,05 à 20% en poids par rapport au poids total de la composition, de préférence à raison de 1 à 10 % en poids.

8. Composition selon l'une quelconque des revendications précédentes, caractérisée par le fait que l'électrolyte est le chlorure de sodium.

9. Composition selon l'une quelconque des revendications précédentes, caractérisées par le fait que le polyacrylamide épaississant est choisi parmi :
- les copolymères d'acrylamide et d'acrylate d'ammonium éventuellement réticulés,
- les copolymères d'acrylamide et de chlorure de méthacryloyloxyéthyl triméthylammonium éventuellement réticulés,
- les copolymères d'acrylamide et d'acide 2-acrylamido 2-méthyl propane sulfonique partiellement ou totalement neutralisé, éventuellement réticulés.

10. Composition selon l'une quelconque des revendications précédentes, caractérisée en ce que le polyacrylamide est présent dans des concentrations comprises entre 0,05 et 5% en poids par rapport au poids total de la composition, de préférence entre 0,2 et 3% en poids.

11. Composition selon l'une quelconque des revendications précédentes, caractérisée par le fait que le rapport pondéral cotensioactif / tensioactif anionique est compris entre 0,01 et 1.

12. Procédé de lavage de la peau ou des fibres kératiniques telles que les cheveux, caractérisé par le fait que l'on applique sur ceux-ci au moins une composition telle que définie dans l'une quelconque des revendications 1 à 11, puis à effectuer un rinçage.

## Patentansprüche

1. Reinigende kosmetische Zusammensetzung, die dadurch gekennzeichnet ist, daß sie mindestens ein anionisches Tensid und mindestens ein nichtionisches oder amphoteres Cotensid in einem Gewichtsverhältnis Cotensid/anionisches Tensid von 1 oder darunter, mindestens ein verdickendes Polyacrylamid und mindestens einen Elektrolyten enthält, der in einer Konzentration von 2 bis 15 Gew.-%, bezogen auf das Gesamtgewicht der Zusammensetzung, enthalten ist und ausgewählt ist unter
i) Lithiumnitrat, Strontiumnitrat, Bariumnitrat, Yttriumnitrat, Neodymnitrat, Gadoliniumnitrat, Mangannitrat, Zinknitrat und Magnesiumnitrat,
ii) Lithiumchlorid, Strontiumchlorid, Bariumchlorid, Yttriumchlorid, Neodymchlorid, Gadoliniumchlorid, Manganchlorid, Zinkchlorid, Magnesiumchlorid und Natriumchlorid,
iii) Calciumsulfat, Strontiumsulfat und Magnesiumsulfat,
iv) Strontiumacetat, Manganacetat und Magnesiumacetat,
wobei die Verbindungen in einer solchen Menge enthalten sind, daß die Zusammensetzung:
(a) ein rheologisches Fließverhalten aufweist, das gekennzeichnet ist durch einen Spannungsbereich, in dem die Viskosität konstant ist, auf den ein Spannungsbereich folgt, in dem die Viskosität abnimmt, wenn die Spannung zunimmt, und
(b) eine Struktur mit lamellarer Phase aufweist, wobei diese Phase imstande ist, nicht wasserlösliche Partikel, die gegebenenfalls in der Zusammensetzung enthalten sind, in Suspension zu halten.

2. Zusammensetzung nach Anspruch 1, dadurch gekennzeichnet, daß die verwendbaren anionischen Tenside ausgewählt sind unter: den Salzen der folgenden Verbindungen: Alkylsulfate, Alkylethersulfate, Alkylamidoethersulfate, Monoglyceridsulfate, Alkylglycerylsulfonate, Alkylsulfonate, Alkylphosphate, Alkylamidsulfonate, Alkylarylsulfonate, α-Olefinsulfonate, Alkylsulfosuccinate, Alkylethersulfosuccinate, Alkylamidsulfosuccinate, Alkylsulfosuccinamate, Alkylsulfoacetate, Alkyletherphosphate, Acylisethionate, N-Acylaminosäuren, wie N-Acylsarcosinate, N-Acylglutamate und N-Acyltaurate, den Salzen von Fettsäuren, wie den Salzen der Undecylensäure, Ölsäure, Ricinoleinsäure, Palmitinsäure, Stearinsäure, den Säuren von Kopraöl oder von hydriertem Kopraöl, den Acylhydroxysäuren, wie Acyllactylate, den Alkyl-D-Galactosid-Uronsäuren und ihren Salzen, den polyethoxylierten Ethercarbonsäuren, insbesondere den polyethoxylierte(n) Ethercarbonsäuren, die 2 bis 24 Ethylenoxid-Einheiten enthalten, und ihren Gemischen.

3. Zusammensetzung nach einem der Ansprüche 1 und 2, dadurch gekennzeichnet, daß die nichtionischen Cotenside ausgewählt sind unter den ethoxylierten, propoxylierten, mit Glycerin veretherten Alkoholen, α-Diolen, Alkylphenolen oder Fettsäuren, wobei diese Verbindungen eine Fettkette enthalten, die 8 bis 28 Kohlenstoffatome aufweist, wobei die Zahl der Ethylenoxid- oder Propylenoxid-Einheiten im Bereich von 1 bis 50 liegen kann und die Zahl der Glycerin-Einheiten insbesondere 1 bis 30 betragen kann, den Ethylenoxid-Propylenoxid-Copolymeren, den Kondensationsprodukten von Ethylenoxid und Propylenoxid mit Fettalkoholen, den polyethoxylierten Fettaminen und Fettamiden, die vorzugsweise 2 bis 30 mol Ethylenoxid enthalten, den mehrfach mit Glycerin veretherten Fettamiden, die im Mittel 1 bis 5 Glycerin-Gruppen enthalten, den mehrfach mit Glycerin veretherten Diglykolamiden, den Estern aus Fettsäuren und Sorbitan, die gegebenenfalls ethoxyliert sind, den Estern aus Fettsäuren und Saccharose, den polyalkoxylierten Fettsäureestern, den gegebenenfalls alkoxylierten Alkylpolyglucosiden, den Alkylglucosidestern, den Derivaten von N-Alkylglucaminen und N-Acylmethylglucaminen, den Aminoxiden und ihren Gemischen.

4. Zusammensetzung nach einem der Ansprüche 1 und 2, dadurch gekennzeichnet, daß die amphoteren Cotenside unter den Derivaten von sekundären oder tertiären aliphatischen Aminen, deren aliphatischer Rest geradkettig oder verzweigt ist und 8 bis 22 Kohlenstoffatome aufweist und die mindestens eine wasserlöslich machende anionische Gruppe, wie eine Carboxy-, Sulfonat-, Sulfat-, Phosphat- oder Phosphonatgruppe, enthalten, ausgewählt sind.

5. Zusammensetzung nach Anspruch 4, dadurch gekennzeichnet, daß die amphoteren Cotenside unter Alkylbetainen, Alkyldimethylbetainen, Alkylsulfobetainen, Alkylamidoalkylbetainen, Alkylamidoalkylsulfobetainen, Imidazolinderivaten, wie den Amphocarboxyglycinat- und den Amphocarboxypropionat-Derivaten, und ihren Gemischen ausgewählt sind.

6. Zusammensetzung nach einem der vorhergehenden Ansprüche, dadurch gekennzeichnet, daß der oder die anionischen Tenside in einem Anteil von 3 bis 50 Gew.-%, vorzugsweise 5 bis 30 Gew.-%, bezogen auf das Gesamtgewicht der Zusammensetzung, enthalten sind.

7. Zusammensetzung nach einem der vorhergehenden Ansprüche, dadurch gekennzeichnet, daß das oder die Cotenside in einem Anteil von 0,05 bis 20 Gew.-%, vorzugsweise 1 bis 10 Gew.-%, bezogen auf das Gesamtgewicht der Zusammensetzung, enthalten sind.

8. Zusammensetzung nach einem der vorhergehenden Ansprüche, dadurch gekennzeichnet, daß es sich bei dem Elektrolyten um Natriumchlorid handelt.

9. Zusammensetzung nach einem der vorhergehenden Ansprüche, dadurch gekennzeichnet, daß das verdickende Polyacrylamid ausgewählt ist unter:
- Acrylamid/Ammoniumacrylat-Copolymeren, die gegebenenfalls vernetzt sind,
- Copolymeren aus Acrylamid und Methacryloyloxyethyltrimethylammoniumchlorid, die gegebenenfalls vernetzt sind,
- Copolymeren aus Acrylamid und 2-Acrylamido-2-methyl-propansulfonsäure, die ganz oder teilweise neutralisiert ist, die gegebenenfalls vernetzt sind.

10. Zusammensetzung nach einem der vorhergehenden Ansprüche, dadurch gekennzeichnet, daß das Polyacrylamid in einer Konzentration von 0,05 bis 5 Gew.-%, vorzugsweise 0,2 bis 3 Gew.-%, bezogen auf das Gesamtgewicht der Zusammensetzung, enthalten ist.

11. Zusammensetzung nach einem der vorhergehenden Ansprüche, dadurch gekennzeichnet, daß das Gewichtsverhältnis Cotensid/anionisches Tensid im Bereich von 0,01 bis 1 liegt.

12. Verfahren zum Waschen der Haut oder von Keratinfasern, wie den Haaren, dadurch gekennzeichnet, daß auf die Haut oder die Keratinfasern mindestens eine Zusammensetzung, die wie in einem der Ansprüche 1 bis 11 definiert ist, aufgetragen wird und anschließend diese Zusammensetzung von der Haut oder den Keratinfasern abgespült wird.

## Claims

1. Detergent cosmetic composition, characterized in that it comprises at least one anionic surfactant, at least one nonionic or amphoteric co-surfactant, in a co-surfactant/anionic surfactant weight ratio less than or equal to 1, at least one thickening polyacrylamide and at least one electrolyte present at a concentration of between 2 and 15% by weight relative to the total weight of the composition, and chosen from:
i) lithium, strontium, barium, yttrium, neodymium, gadolinium, manganese, zinc or magnesium nitrate,
ii) lithium, strontium, barium, yttrium, neodymium, gadolinium, manganese, zinc, magnesium or sodium chloride,
iii) calcium, strontium or magnesium sulphate,
iv) strontium, manganese or magnesium acetate,
the said compounds being in an amount such that the composition has:
(a) a rheological flow behaviour characterized by a stress range for which the viscosity is constant, followed by a stress range for which the viscosity decreases as the stress increases, and
(b) a lamellar phase structure, this phase being capable of maintaining in suspension water-insoluble particles which may be present in the composition.

2. Composition according to Claim 1, characterized in that the anionic surfactants which can be used are chosen from the salts of the following compounds: alkyl sulphates, alkyl ether sulphates, alkylamidoether sulphates, monoglyceride sulphates, alkylglyceryl sulphonates, alkyl sulphonates, alkyl phosphates, alkylamide sulphonates, alkylaryl sulphonates, α-olefin sulphonates, alkyl sulphosuccinates, alkyl ether sulphosuccinates, alkylamide sulphosuccinates, alkyl sulphosuccinamates, alkyl sulphoacetates, alkyl ether phosphates, acyl isethionates and N-acylamino acids such as N-acylsarcosinates, N-acylglutamates and N-acyltaurates, fatty acid salts such as the salts of undecylenic, oleic, ricinoleic, palmitic and stearic acids, coconut oil acid or hydrogenated coconut oil acid, acylhydroxy acids such as acyllactylates, alkyl-D-galactosiduronic acids and salts thereof, polyoxyalkylenated carboxylic acid ethers, in particular those containing from 2 to 24 ethylene oxide groups, and mixtures thereof.

3. Composition according to either of Claims 1 and 2, characterized in that the nonionic co-surfactants are chosen from ethoxylated, propoxylated or glycerolated fatty acids, alkylphenols, alpha-diols or alcohols, these compounds having a fatty chain containing from 8 to 28 carbon atoms, it being possible for the number of ethylene oxide or propylene oxide groups to range from 1 to 50 and the number of glycerol groups to range in particular from 1 to 30, copolymers of ethylene oxide and propylene oxide, condensates of ethylene oxide and propylene oxide with fatty alcohols, polyethoxylated fatty amines or amides preferably having from 2 to 30 mol of ethylene oxide, polyglycerolated fatty amides containing on average 1 to 5 glycerol groups, polyglycerolated diglycolamides, optionally oxyethylenated fatty acid esters of sorbitan, fatty acid esters of sucrose, polyoxyalkylenated fatty acid esters, optionally oxyalkylenated alkylpolyglycosides, alkylglucoside esters, N-alkylglucamine and N-acylmethylglucamine derivatives, amine oxides and mixtures thereof.

4. Composition according to either of Claims 1 and 2, characterized in that the amphoteric co-surfactants are chosen from secondary or tertiary aliphatic amine derivatives in which the aliphatic radical is a linear or branched chain containing 8 to 22 carbon atoms and containing at least one anionic water-solubilizing carboxylate, sulphonate, sulphate, phosphate or phosphonate group.

5. Composition according to Claim 4, characterized in that the amphoteric co-surfactants are chosen from alkylbetaines, alkyldimethylbetaines, alkylsulphobetaines, alkylamidoalkylbetaines, alkylamidoalkylsulphobetaines, imidazoline derivatives such as amphocarboxyglycinate or amphocarboxypropionate derivatives, and mixtures thereof.

6. Composition according to any one of the preceding claims, characterized in that the anionic surfactant(s) are present in a proportion of 3 to 50% by weight relative to the total weight of the composition, preferably in a proportion of 5 to 30% by weight.

7. Composition according to any one of the preceding claims, characterized in that the co-surfactant(s) are present in a proportion of 0.05 to 20% by weight relative to the total weight of the composition, preferably in a proportion of 1 to 10% by weight.

8. Composition according to any one of the preceding claims, characterized in that the electrolyte is sodium chloride.

9. Composition according to any one of the preceding claims, characterized in that the thickening polyacrylamide is chosen from:
- optionally crosslinked copolymers of acrylamide and ammonium acrylate,
- optionally crosslinked copolymers of acrylamide and methacryloyloxyethyltrimethylammonium chloride,
- optionally crosslinked and partially or totally neutralized copolymers of acrylamide and 2-acrylamido-2-methylpropanesulphonic acid.

10. Composition according to any one of the preceding claims, characterized in that the polyacrylamide is present in concentrations of between 0.05 and 5% by weight relative to the total weight of the composition, preferably between 0.2 and 3% by weight.

11. Composition according to any one of the preceding claims, characterized in that the co-surfactant/anionic surfactant weight ratio is between 0.01 and 1.

12. Process for washing the skin or keratin fibres such as the hair, characterized in that at least one composition as defined in any one of Claims 1 to 11 is applied to them and rinsing is then carried out.
